(19) Europäisches Patentamt
European Patent Office
Office européen des brevets

(11) Numéro de publication : **0 215 824**
**B1**

(12) # FASCICULE DE BREVET EUROPÉEN

(45) Date de publication du fascicule du brevet : **03.01.90**

(21) Numéro de dépôt : **86901326.8**

(22) Date de dépôt : **10.03.86**

(86) Numéro de dépôt international : **PCT/CH 86/00032**

(87) Numéro de publication internationale : **WO/8605517 (25.09.86 Gazette 86/21)**

(51) Int. Cl.[5] : **C 12 Q 1/00**, C 12 Q 1/28, C 12 Q 1/54, C 12 Q 1/58, C 12 Q 1/32

(54) **PROCEDE ANALYTIQUE POUR LA DETERMINATION D'UN COENZYME REDUIT.**

(30) Priorité : **15.03.85 CH 1165/85**

(43) Date de publication de la demande : **01.04.87 Bulletin 87/14**

(45) Mention de la délivrance du brevet : **03.01.90 Bulletin 90/01**

(84) Etats contractants désignés : **CH DE FR GB IT LI NL SE**

(56) Documents cités :
**EP-A- 0 029 104**
**EP-A- 0 124 909**
**WO-A-00 /108 1**
**GB-A- 1 192 046**
**Clinical Chemistry, vol. 28, no. 9, September 1982, Easton, PA. (US); I.W. Siddiqi:"An electro-chemical assay system for peroxidase and peroxidase-coupled reactions bases on a fluoride ion-selective electrode", pages 1962-1967**
**Chemical Abstracts, vol. 99, no. 11, 12 September 1983, Columbus, Ohio (US); N. Figuerona et al.: "NMR study of slowly exchanging imino protons in yeast tRNA.Asp", p. 209, abrégé 83902p, & Proc.Natl. Acad. Sci. USA, vol.80, no.14, 1983, 4330-4333**

(73) Titulaire : **BATTELLE MEMORIAL INSTITUTE**
**7 route de Drize**
**CH-1227 Carouge/Genève (CH)**

(72) Inventeur : **SIDDIQI, Iqbal**
**5, Tour de Champel**
**CH-1206 Genève (CH)**
Inventeur : **BROCHOT, Jean**
**Lagnellue Feigères**
**F-74160 St. Jullen-en-Genevois (FR)**

(74) Mandataire : **Dousse, Blasco et al**
**7, route de Drize**
**CH-1227 Carouge/Genève (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

## Description

La présente invention se rapporte au domaine des analyses biologiques et, plus particulièrement à la détermination de co-enzymes dont la molécule contient de la nicotinamide, notamment NADH, NADPH et APADH.

On sait que les abréviations ci-dessus désignent les corps suivants :

NADH = forme réduite du NAD (ou $NAD^+$) (nicotinamide-adenine-dinucléotide), appelé aussi co-enzyme I ou DPN (diphosphopyridine nucléotide)

NADPH = forme réduite du NADP (nicotinamide-adenine-dinucléotide phosphate) appelé aussi co-enzyme II ou TPN (triphosphopyridine nucléotide)

APADH = forme réduite de l'APAD (acétylpyridine-adenine-dinucléotide).

La structure du NAD est composée successivement d'un groupe 3-amidopyridinium fixé en position 1- d'une unité ribose, elle-même reliée par sa position -5- à un groupe diphosphate fixé en -5'- d'une seconde unité ribose laquelle comporte en -1'- un groupe adenine. La conversion réversible du NAD en NADH est schématisée comme suit (R désignant la chaîne ribose-diphosphate-ribose-adenine) :

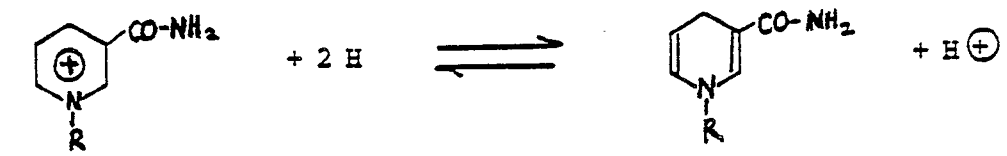


$NAD^+$ NADH

Les co-enzymes à nicotinamide jouent un rôle dans un grand nombre de réactions enzymatiques biochimiques utilisées comme test cliniques. Parmi celles-ci, on peut citer notamment l'oxydation des α-hydroxyacides en acides cétoniques correspondants en présence d'une déshydrogénase appropriée. A titre d'exemple on peut citer l'oxydation de l'acide lactique ou des lactates en acide pyruvique

$$CH_3\text{—}CHOH\text{—}COOH + NAD \xrightarrow[\text{déshydrogénase}]{\text{lactate}} CH_3CO\text{—}COOH + NADH$$

De manière analogue le glucose-6-phosphate est oxydé en présence de NADP et de glucose-6-phosphate déshydrogénase (G6PDH) en glucono-δ-lactone-6-phosphate et NADPH. Le dosage du NADP ou du NADPH dans une telle réaction revêt une grande importance car, indirectement, il permet de déterminer le glucose dans les fluides biologiques, celui-ci étant, auparavant, transformé en glucose-6-phosphate en présence d'ATP (adenosine triphosphate) et d'hexokinase.

Par ailleurs, le NADH agit comme facteur co-enzymatique dans la conversion par les sels d'ammonium du 2-oxoglutarate en L-glutamate en présence de GLDH (glutamate déshydrogénase) ce qui permet, par détermination du NADH restant (ou du $NAD^+$ formé), de doser l'ammonium du milieu réactionnel. Cette réaction est applicable pour le dosage de l'urée dans les fluides biologiques, celle-ci fournissant, en présence d'uréase, le $NH_3$ impliqué en tant qu'ion $NH_4$ dans la conversion susindiquée.

On trouvera description d'autres applications liées à la détermination des facteurs $NAD^+$, NADH et APADH dans les documents suivants : EP-A-29 104 (MILES) ; FR-A-2 299 644 (AKZO).

La détermination des co-enzymes ci-dessus, suivant l'un ou l'autre de leurs états d'oxydoréduction revêtant une grande importance, de nombreuses techniques ont été proposées à cet effet.

Ainsi, le NAD et le NADH ayant des absorptions différentes dans l'UV, on peut doser une forme en présence de l'autre par spectrophotométrie. On peut, par ailleurs, amplifier la sensibilité des déterminations spectrophotométriques par utilisation conjointe de composés rédox colorés, par exemple des composés de tetrazolium qui, en présence de NADH ou de NADPH et d'un accepteur d'électrons comme le méthosulphate de phénazine, donne des sels de formazan fortement colorés. (Voir par exemple le document EP-A-114 267). On peut également utiliser des techniques fluorimétriques telles que décrites, par exemple, dans le document FR-A-2 266 644.

On peut également procéder par voie électrochimique tel que décrit dans le document J 56 035 050 où on oxyde le NADH ou le NADPH par du bleu de Meldola, puis on oxyde électrochimiquement la forme réduite de ce colorant et on mesure le courant d'oxydation.

On a récemment préconisé (EP-A-29 104) de faire intervenir la réaction suivante :

$$NADH \text{ (ou NADPH)} + O_2 \xrightarrow[\text{pseudosubstrat}]{\text{hydroxylase}} NAD \text{ (ou NADP)} + H_2O_2$$

Le peroxyde d'hydrogène ainsi libéré est ensuite dosé par les moyens habituels, par exemple par son action, catalysée par la peroxydase, sur un indicateur rédox, la forme oxydée de celui-ci étant dosée par colorimétrie.

Le document EP-A-124 909 décrit un procédé similaire mais simplifié, car ne faisant intervenir comme enzyme que la peroxydase seule. Le procédé consiste à faire réagir le coenzyme avec la peroxydase en présence d'ions métalliques tels que $Mn^{+2}$ ou $Co^{+2}$ ce qui engendre la formation quantitative de peroxyde d'hydrogène, ce dernier étant alors dosé comme ci-dessus par les moyens habituels, par exemple par colorimétrie, notamment le système comprenant la 4-aminoantipyrine comme coupleur et un composé phénolique ou une amine aromatique comme chromophore. En l'absence d'ions métalliques (voir p. 5, paragraphe 1 de ce document) la formation de $H_2O_2$ n'est pas quantitative et, en conséquence, la réaction n'a pas d'application analytique.

Quoique les techniques colorimétriques soient très séduisantes, elles ne sont applicables qu'en milieu non coloré et optiquement transparent ce qui est loin d'être le cas de la plupart des fluides biologiques à analyser. Par ailleurs, elle est relativement compliquée à appliquer et ne présente en général pas la sensibilité des techniques électrométriques. A ce sujet, la présente requérante a récemment divulgué (voir EP-A-20 623) que dans l'analyse de $H_2O_2$ engendré par oxydation du glucose en présence de glucose oxidase, la technique consistant à faire agir cet $H_2O_2$ sur un composé aromatique fluoré en présence de peroxydase de manière à libérer des ions fluorure, ceux-ci étant ensuite déterminés électrométriquement au moyen d'une électrode spécifique de ces ions, conduit à d'excellents résultats tant au point de vue de la sensibilité que de la précision des mesures.

Il devenait dès lors tentant de combiner la réaction de formation de $H_2O_2$ décrite dans le document EP-A-124 909 avec la détermination fluorométrique de $H_2O_2$ décrite dans le document EP-A-20 623. Or, c'est avec surprise qu'on a constaté qu'une telle combinaison n'est pas directement possible en raison des observations suivantes :

(1) En présence d'ions métalliques en concentration telle que celle préconisée dans le document EP-A-124 909 (5 mmole/l ; p. 9, ligne 6), l'activité de la peroxydase est normalement inhibée en ce qui concerne son action catalytique sur la scission de la liaison C—F et il est nécessaire de neutraliser cet effet inhibiteur par adjonction d'un activateur, tel que la 4-aminoantipyrine.

(2) En l'absence d'ions métalliques, ou tout au moins en présence de très faibles quantités de ceux-ci (d'un ordre de grandeur inférieur de 1000 aux quantités préconisées dans le document EP-A-124 909, ou moins, c'est-à-dire dans des conditions où il a été admis que $H_2O_2$ ne se forme pas quantitativement), on a découvert avec surprise que les coenzymes NADH ou NADPH pouvaient bel et bien être dosés quantitativement par la réaction de scission de la liaison C—F en fluorure en présence de peroxydase (et ceci sans apport d'antipyrine dont la présence, dans ce cas, a, bien au contraire, un effet inhibiteur). Il s'agit donc là d'un type de réaction nouveau ne faisant pas intervenir la formation quantitative intermédiaire d'eau oxygénée comme dans l'art antérieur. Cet état de choses a d'ailleurs été confirmé par adjonction au milieu d'analyse d'une enzyme telle que la catalase qui provoque la consommation de $H_2O_2$ au fur et à mesure de sa formation. Ainsi, l'adjonction de catalase au milieu réactionnel préconisé par le document EP-A-124 909 ([Mn + 2] ~ $10^{-2}$ M) provoque une chute d'efficacité en ce qui concerne la scission de la liaison carbone-fluor de l'ordre de 70 % alors que dans un milieu correspondant, mais a $10^{-5}$ M de Mn + 2, l'effet de la catalase est négligeable.

Les découvertes ayant fait l'objet des considérations ci-dessus ont donc permis l'application d'une technique nouvelle et imprévue à la détermination des co-enzymes à nicotinamide en général. Cette technique a donné lieu au procédé de la présente invention tel que défini à la revendication 1 annexée. Un des avantages de ce procédé est la faculté de doser les co-enzymes en présence de réactifs inhibiteurs de la formation d'$H_2O_2$. Par substances à activité peroxidasique, on entend comprendre toutes les substances ayant des propriétés catalytiques similaires à celles de la peroxydase, notamment l'hémoglobine et ses dérivés (voir Boyer et al., « The Enzymes » 8 (1963) Academic Press).

Bien entendu, pour la détermination de l'ion fluorure libéré par ce procédé on aura, de préférence, recours à une méthode électrométrique telle que divulguée dans le document EP-A-20 623. Cependant, toute autre technique de détermination du fluorure pourrait également convenir.

Le principe général du présent procédé peut être décrit brièvement comme suit : le système de base de l'invention consiste à ajouter à un milieu tampon contenant le co-enzyme réduit à doser un excès d'un composé fluoré aromatique et de peroxydase puis d'agiter ce milieu en présence d'air. Il se produit alors une scission de la liaison FC du composé fluoré et libération correspondante d'ions $F^-$ à une vitesse réactionnelle proportionnelle à la quantité de co-enzyme présent. On notera qu'il existe également un facteur de stoechiométrie entre cette quantité d'ions fluorure libérés et la quantité de co-enzyme à doser ; cependant, au point de vue analytique, cette relation est moins intéressante car la réaction, de vive au départ, se ralentit fortement par la suite et il n'est pas pratique de procéder à des mesures du type « end-point ». On pourrait cependant prévoir des analyses à « temps fixé » en procédant à un dosage des réactants après un temps donné, toujours le même dans une série d'analyses du même type. Cependant, en général, il est préférable de procéder aux mesures de vitesses de libération des ions $F^-$ dans des conditions bien standardisées afin d'assurer une bonne reproductibilité des mesures ; notamment, la mesure des pentes des courbes de vitesse (qui bien entendu dépendent de certains paramètres réactionnels aussi bien que de la concentration de la substance à mesurer) se fera avantageusement

après un certain temps de latence (incubation), ce temps étant conservé fixe pour une série d'essais comparatifs. On notera cependant que le temps de latence peut être légèrement différent d'une analyse à l'autre, la vitesse maximale étant d'autant plus tôt atteinte que la concentration en NADH est plus grande. Comme tampons, on peut utiliser les tampons usuels à pH de 5 à 7,5, notamment acétate, « tris », cacodylate, etc... On préfère ce dernier car il permet une rapide stabilisation de réponse de l'électrode à fluor. Par ailleurs, on a remarqué que la vitesse réactionnelle (la scission de la liaison C—F) dépend de la concentration du tampon en diméthylarsinate. De préférence, le tampon cacodylate présente une molarité de 0,05 M à 0,5 M.

La détermination de la concentration des ions $F^-$ libérés sera effectuée de préférence au moyen d'une électrode sensible aux ions $F^-$ mais inerte aux autres types d'ions. Comme type d'électrode, on peut favorablement utiliser une électrode sélective des ions $F^-$ de type 96-09 provenant de ORION RESEARCH INC. Cambridge, Mass. USA. Cependant, d'autres électrodes peuvent également convenir. On trouvera tous les détails relatifs à l'utilisation de telles électrodes pour le dosage des ions $F^-$ dans le document précité EP-A-20 623. Il est bien entendu que le présent procédé s'applique également à la détermination de tous constituants additionnels susceptible de réagir quantitativement en présence des co-enzymes à nicotinamide (sous forme réduite ou oxydée) et, par là, d'entraîner une variation (mesurable par le présent procédé) de la forme réduite existant dans le milieu.

Ainsi, lorsque le présent milieu réactionnel est mis en œuvre dans la détermination de systèmes précurseurs, c'est-à-dire ceux où la formation quantitative de co-enzyme dépend d'une ou plusieurs transformations successives d'une substance à mesurer, la technique à appliquer est tout à fait similaire à celle décrite ci-dessus ; en effet, et c'est là un des avantages significatifs de l'invention, la détection et la mesure électrochimique des ions fluorures est indifférente à la présence, dans le milieu réactionnel, de nombreux autres facteurs et substances dissous. Aussi, le présent procédé est-il directement applicable à la mesure du glucose et de l'urée suivant des schémas similaires à ceux cités dans l'introduction.

Ainsi, pour la détermination du glucose, on fera d'abord intervenir sa transformation en glucose-6-phosphate en présence d'ATP et d'hexokinase (ou d'une autre enzyme de propriétés similaires) ; puis on ajoutera une quantité connue de NADP et on mesurera, suivant le processus précité, le NADPH formé lors de la transformation du glucose-6-phosphate en glucono-δ-lactone-6-phosphate en présence de G6PDH. Au départ de la réaction, le milieu réactionnel ne contient pas de NADH, ce dernier apparaissant au cours du processus enzymatique. En fait, les deux réactions, celle catalysée par l'hexokinase et celle catalysée par la G6, se déroulent simultanément en présence de $NAD^+$ et d'ATP. La formation de NADH est donc mesurée, en continu, depuis le début de la réaction.

Des considérations voisines s'appliquent à tous les autres cas de réaction biochimique faisant intervenir les présents co-enzymes soit à titre de produits de départ, soit à titre de produits réactionnels. C'est ainsi le cas du dosage de l'urée.

A un échantillon de celle-ci, prélevé dans un fluide biologique, par exemple l'urine ou le plasma sanguin et mélangé à un tampon approprié, on ajoute un excès d'uréase, d'oxoglutarate et de GLDH accompagnés du composé fluoré et d'une quantité exactement connue de NADH (en excès, elle aussi, mais cependant du même ordre de grandeur que celle de l'urée à mesurer pour ne pas se heurter à des problèmes de disproportions). Puis, après avoir attendu un temps déterminé pour que s'effectue la transformation du NADH et $NAD^+$, on introduit l'électrode à ions $F^-$ dans le mélange et met celui-ci en agitation à l'air ; puis, on ajoute une quantité catalytique de peroxydase (POD) et on mesure la variation du potentiel de l'électrode au cours du temps, cette mesure permettant de doser la quantité de NADH non utilisée dans la réaction et, donc, d'en déduire la quantité d'urée de l'échantillon analytique.

On a fourni en détail dans le document EP-A-20 623 la technique opératoire impliquant l'électrode à fluor ainsi que les considérations physicochimiques relatives à l'interprétation des résultats.

En bref, pour déterminer la vitesse de libération des ions $F^-$ consécutive à la réaction d'un échantillon inconnu, on se réfère de préférence à une courbe étalon. On peut obtenir une courbe d'étalonnage en dosant, comme décrit plus haut, une série d'échantillons à concentration connues de co-enzyme. On enregistre, pour chaque échantillon, la vitesse de libération des $F^-$ et on mesure la pente des courbes de cinétique à un moment (qui est bien entendu le même pour chaque échantillon) où les courbes de vitesse sont à peu près des droites. Puis on reporte graphiquement ces valeurs de pente en fonction des concentrations en co-enzyme, de manière à établir une courbe de référence standard. Les paramètres électrométriques mesurés à utiliser pour la préparation des courbes de cinétiques peuvent être les lectures de tensions du système électrométrique utilisé conjointement avec l'électrode à fluor (mV) ou, mieux, les valeurs correspondantes de $[F^-]$ calculables par l'équation de Nernst, laquelle, dans ce cas, a la forme suivante :

$$E = E' - S \cdot \mathrm{Log}\,[F^-]$$

où E est le voltage mesuré et E' est une constante propre du système qu'on détermine expérimentalement et qui englobe les facteurs d'activité et les potentiels de jonctions liquides. S est la « pente de Nernst » qui est une constante égalant environ 57,5 mV (dans le tampon cacodylate à pH 7,5) pour une variation de 10 unités de la concentration des $F^-$, cette dernière étant exprimée en moles/l. Si les valeurs de $[F^-]$ calculées à partir de la relation ci-dessus sont utilisées dans les graphiques de vitesse au lieu des valeurs

en mV, on obtient des courbes se rapprochant plus de lignes droites, dont la pente est plus facile à établir et qui permettent de dessiner des graphiques de référence plus précis.

Parmi les composés aromatiques fluorés sur le noyau convenant à la mise en œuvre de la présente invention, on cite, plus particulièrement, les 2-, 3- et 4-fluorophénols, le tetrafluorophénol, le pentafluorphénol et la p-fluoroaniline ; on préfère utiliser le 4- et le 2-fluorophénol.

On notera par ailleurs que le taux minimum de co-enzyme détectable et mesurable par ce procédé peut être abaissé en ajoutant une faible quantité d'ions manganèse II au milieu réactionnel. Cette addition n'est cependant valable que dans les cas où la concentration en co-enzyme est très faible et au voisinage ou en dessous de la limite inférieure normalement accessible sans manganèse. Ainsi, l'adjonction de $Mn^{++}$ (concentration de $2 \times 10^{-6}$ à $10^{-4}$ M) est utile lorsque les quantités de co-enzyme sont de l'ordre $10^{-4}$ à $10^{-5}$ M. Dans un tel cas, on arrive à multiplier la vitesse pratique de la réaction par un facteur d'ordre 5. En présence de concentration plus élevée de co-enzyme, la présence d'ions $Mn^{+2}$ s'accompagne d'un défaut de linéarité tendant à diminuer la précision de la mesure ; en conséquence, lorsque les concentrations de co-enzyme à déterminer sont supérieures à $10^{-4}$, il peut être préférable de ne pas utiliser de manganèse.

Les exemples suivants, pour la meilleure compréhension desquels on se référera au dessin annexé, illustrent l'invention plus en détail.

La fig. 1 est un graphique représentant la variation de la quantité de NADH en fonction de la vitesse de libération des ions $F^-$ à pH 7,5.

La fig. 2 est un graphique similaire à celui de la fig. 1, mais se rapportant à l'analyse du glucose.

La fig. 3 est un graphique similaire à celui de la fig. 1, mais se rapportant à l'analyse de l'urée.

La fig. 4 est un graphique similaire à celui de la fig. 1 mais se rapportant à un pH de 5,5.

## Exemple 1

Détermination du NADH à pH 7,5

Pour cette détermination suivant l'invention, on travaille dans une solution tampon. De préférence, on utilise le tampon cacodylate classique. Les tampons phosphates de pH 7-7,5 conviennent également mais, dans ce cas, la stabilisation de l'électrode de mesure (lorsqu'on détermine $F^-$ électrométriquement) se fait plus difficilement et la calibration au moyen de solutions connues de NaF est moins précise et reproductible. Le tampon « tris » entre 7 et 7,5 convient également.

a) Préparation du tampon cacodylate : dans 800 ml d'eau deux fois distillée, on a dissous 21,41 g de diméthylarsinate de sodium-trihydrate (MERCK). On a ensuite ajouté 1 ml d'une solution $5 \times 10^{-3}$ M de chlorure de manganèse (II) et 2 ml d'une solution $10^{-3}$ M de NaF. On a ensuite porté le pH à exactement 7,5 par du HCl N et on a complété le niveau à 1 litre avec de l'eau deux fois distillée.

b) Milieu réactionnel pour l'analyse : on a dissous 0,112 g de p-fluorophénol (EGA-CHEMIE) dans 100 ml du tampon cacodylate (a) ci-dessus. Le p-fluorophénol contenant toujours une faible quantité de $F^-$, la concentration d'ion fluorure dans le milieu (b) est de l'ordre de 3-5 $\times 10^{-6}$ M.

c) Solution de peroxydase : on a utilisé de la peroxydase de raifort (BOEHRINGER ; RZ 3,0 ; 250 U/mg) ; on en a dissous dans le tampon (a) ci-dessus une quantité convenable pour réaliser une solution à environ 0,4 g/l (100 U/ml).

d) Solutions d'étalonnage de NADH : on a utilisé le sel disodique du NADH (SIGMA) de poids moléculaire théorique 759 D. On en a dissous dans le tampon cacodylate (a) de manière à réaliser une solution titrée de 38 g/l ($5 \times 10^{-2}$ M). Puis, par dilution au moyen du même tampon, on a préparé des solutions d'étalonnage à 3,75 ; 2,25 et 1,25 $\times 10^{-2}$ M ; 5 et 1,25 $\times 10^{-3}$ M ; et $5 \times 10^{-4}$ M.

e) Analyse : comme récipient laboratoire, on a utilisé un becher de 10 ml en polypropylène agité magnétiquement. Dans ce becher, on a placé 4,8 ml de milieu réactionnel (b), puis 0,1 ml de solution (c) ; la solution à concentration zéro de NADH est constituée par le tampon (a) seul. On a plongé dans le milieu une électrode spécifiquement sensible aux ions $F^-$ (type Orion 96-09 ; Orion RESEARCH, Cambridge, Mass., USA comportant sa propre électrode de référence), celle-ci étant reliée à un électromètre de type habituel (KEITHLEY Electrometer, Cleveland, Ohio, USA).

L'appareil étant enclenché, on a laissé le tout se stabiliser 3 minutes à température ambiante sous agitation puis on a ajouté 0,1 ml d'une des solutions standard de NADH (d). On a alors procédé à la mesure de la vitesse de libération des ions $F^-$ pendant une période d'environ 3 min par la réaction précitée en enregistrant, au moyen d'un recorder, le potentiel d'électrode correspondant. Par ailleurs, les données ainsi enregistrées sont transmises à un calculateur qui fournit la pente de la courbe de vitesse dans sa région la plus linéaire (après 20 sec environ et pendant environ 30 sec à 1 min., ces paramètres étant, bien entendu, gardés constants pour la phase totale d'étalonnage ainsi que pour les analyses de solutions inconnues faites par la suite).

Les valeurs trouvées (S), exprimées directement en µMole de $F^-$ libérés par min à partir de la formule précitée, figurent ci-dessous :

| Concentration de NADA (μMoles) | Pente de la courbe de vitesse (S) μM (F⁻)/min |
|---|---|
| 0 | 0 |
| 10 | 0,236 |
| 25 | 0,512 |
| 100 | 2,058 |
| 250 | 3,498 |
| 500 | 5,976 |
| 750 | 7,581 |
| 1000 | 8,082 |

Le graphique obtenu grâce aux valeurs résumées ci-dessus est représenté à la fig. 1.

Pour l'analyse de solutions inconnues de NADH, on a procédé exactement comme décrit ci-dessus et, après avoir déterminé la valeur de la pente de la courbe réactionnelle, on a obtenu la valeur recherchée grâce au graphique étalon de la fig. 1. Bien entendu, dans la pratique des analyses chimiques, ces résultats sont fournis directement par le calculateur.

Exemple 2

Détermination du NADH à pH 5,5

On a procédé comme dans l'Exemple 1 avec, cependant, les différences suivantes :

a) Le tampon cacodylate (0,1 M) est identique à celui de l'Exemple 1 à la différence près que son pH a été ajusté à 5,5 avec du HCl N.

b) Le milieu réactionnel contient 0,01 M de 4-fluorophénol 4FP.

c) Solution de peroxidase : solution à 300 U/ml (env. 1,2 g/l).

d) Solution d'étalonnage : ces solutions sont calculées de manière que 0,1 ml (prise) contienne, successivement, les quantités de NADH suivantes (en μM) : 0, 25, 50, 75, 100, 125, 150, 175, 200.

L'analyse a été effectuée comme décrit à l'Exemple 1. La réaction est cependant plus rapide et la pente effective de la courbe de vitesse est remarquablement constante, déjà 30 sec après l'addition du NADH. Les résultats figurent ci-dessous ainsi qu'à la fig. 4.

| [NADH] (μMole) | Vitesse ([F⁻])/μM/l/min |
|---|---|
| 0 | 0 |
| 25 | 2,04 |
| 50 | 4,41 |
| 75 | 6,74 |
| 100 | 8,80 |
| 125 | 11,28 |
| 150 | 13,33 |
| 175 | 15,45 |
| 200 | 18,01 |

Exemple 3

Détermination du glucose

Cette détermination est effectuée selon le schéma suivant (voir Notice : Test Glucose Rapide, ROCHE, DIAGNOSTICA) :

$$\text{D-Glucose} + \text{ATP} \xrightarrow{\text{HK}} \text{glucose-6-phosphate} + \text{ADP}$$

$$\text{Glucose-6-phosphate} + \text{NAD}^+ \xrightarrow{\text{G6P-DH}} \text{D-Gluconate-6-phosphate} + \text{NADH} + \text{H}^+$$

ATP = adenosine triphosphate
HK = hexokinase
ADP = adenosine diphosphate
G6P-D = glucose-6-phosphate deshydrogénase

a) Tampon cacodylate : Il s'agit du même tampon qu'à l'exemple 1 précédent.

b) Milieu réactionnel : Dans 24,5 ml de tampon (a), on a dissous 1 dose de produit No 0711004 livré par la Société ROCHE et contenant : ATP > 50 µmoles ; $NAD^+$ > 50 µmoles ; HK > 7 U ; G6P-DH > 8 U. On a ensuite ajouté 0,5 ml de la solution (c) de peroxydase de l'exemple 1 et 28 mg de p-fluorophénol. On obtient ainsi une solution dans laquelle les réactifs ont les concentrations suivantes : ATP $2 \times 10^{-3}$ M ; $NAD^+$ $2 \times 10^{-3}$ M ; HK 0,28 U/ml ; G6P-DH 0,32 U/ml.

c) Solution de glucose standard ; on a utilisé des solutions à 0,5 ; 1 ; 2 ; 3 ; 4 et 5 g/l dans de l'acide benzoïque aqueux à 0,1 %.

Pour l'analyse, on a procédé comme décrit à l'exemple 1 en utilisant 4,9 ml du milieu réactionnel et 0,1 ml de la solution étalon (c) ajoutée après une période de stabilisation de 3 min. Les résultats obtenus figurent ci-dessous ainsi qu'à la fig. 2.

La courbe représentée à la fig. 2 a permis, par comparaison, de mettre en relation les valeurs de cinétique obtenues avec des solutions inconnues de glucose avec la concentration effective en glucose de ces solutions.

| Concentration en glucose (µMoles) | Vitesse (µMoles $F^-$)/l/min |
|---|---|
| 0 | 0,09 |
| 55,5 | 0,50 |
| 111 | 0,69 |
| 222 | 1,08 |
| 333 | 1,29 |
| 444 | 1,50 |
| 555 | 1,65 |

Exemple 4

Détermination de l'urée.

Cette réaction est basée sur le schéma suivant (voir « Urea UV Test de ROCHE DIAGNOSTICA) :

$$\text{urée} + 2\,H_2O \xrightarrow{\text{uréase}} 2\,NH_3 + CO_2$$

$$NH_3 + H^+ \longrightarrow NH_4^+$$

$$NH_4^+ + \text{2-oxoglutarate} + NADH \xrightleftharpoons{GLDH} \text{L-Glutamate} + NAD^+ + H_2O$$

GLDH = glutamate déshydrogenase.

(a) Tampon cacodylate : même tampon (a) qu'à l'exemple 1.

(b) Milieu réactionnel : dans 30 ml de tampon (a), on a dissous 1 dose de mélange enzyme-substrat de produit No 0713228 livré par la Société ROCHE (Reagent kit for the kinetic determination of urea in serum). Cette dose contient : urease > 25 U ; 2-oxoglutarate 197 µmole ; NADH 6 µmole. On a encore ajouté 33,7 mg de p-fluorophénol et 0,2 ml de solution de GLDH (~ 50 U). Le milieu (b) contient donc les réactifs ci-dessus aux concentrations suivantes :

Urease 0,83 U/ml ; 2-oxoglutarate $6,57 \times 10^{-3}$ M ; NADH $2 \times 10^{-4}$ M ; GLDH 3,33 U/ml.

(c) Solution de peroxydase : il s'agit de la même solution à 100 U/ml décrite à l'exemple 1.

(d) Solution d'étalonnage d'urée : on a préparé des solutions d'urée dans un tampon phosphate physiologique, pH 6,7 aux concentrations suivantes :

7,13 ; 4,99 ; 3,56 ; $1,426 \times 10^{-3}$ M

Pour l'analyse, on a procédé comme à l'exemple 1 en utilisant 3,82 ml de milieu réactionnel (b) et 0,1 ml de la solution d'urée étalon. On a agité le mélange des réactifs à l'exception de la peroxydase (l'électrode étant mise en service) pendant 10 min, puis on a ajouté 0,08 ml de la solution de peroxydase. On a alors fait débuter la mesure et effectué le calcul des vitesses après un délai de 20 à 30 sec. Les résultats figurent ci-dessous ainsi que sur le graphique de la fig. 3.

On notera que dans ce test, on dose le NADH qui apparaît au cours de la réaction ; c'est la raison pour laquelle on prévoit un temps d'incubation de 10 min ; on a constaté que l'analyse est toujours valable quoique moins performante avec des temps d'incubation de seulement 3 min.

| Concentration d'urée de la solut. standard (10-3M) | Quantité d'urée dans la mesure (χ x 10-6Mole | Vitesse (µmoles F-/l/min) |
|---|---|---|
| 0 | 0 | 0,91 |
| 1,426 | 35,6 | 0,81 |
| 3,56 | 89 | 0,67 |
| 4,99 | 125 | 0,58 |
| 7,13 | 178 | 0,46 |

Le graphique de la fig. 3 a été utilisé pour déterminer, par comparaison, la concentration en urée d'échantillons inconnus, ceux-ci ayant été soumis à la procédure d'analyse décrite ci-dessus.

Exemple 5

Détermination du NADH en présence d'organofluorés divers.

(a) On a utilisé un tampon cacodylate identique au tampon (a) de l'exemple 1.

(b) On a préparé une série de milieux réactionnels en procédant comme décrit pour la solution (b) de l'exemple 1, de manière à réaliser des solutions à $10^{-2}$ M dans le tampon (a) des organofluorés suivants : 4-fluorophénol (4FP) ; 3-fluorophénol (3FP) ; 2-fluorophénol (2FP) ; tetrafluorophénol (TFP) ; pentafluorophénol (PFP).

(c) On a utilisé une solution de peroxydase identique à la solution (c) de l'exemple 1.

(d) On a utilisé une solution de NADH (voir solution (d) de l'exemple 1) de $5 \times 10^{-2}$ M dans le tampon (a).

On a procédé à l'analyse comme décrit à l'exemple 1 avec des quantités identiques de réactifs ; milieu réactionnel (b) 4,8 ml ; solution (c) 0,1 ml solution (d) 0,1 ml.

Les résultats des mesures de vitesses figurent ci-dessous :

| Organofluoré | µMF⁻/min |
|---|---|
| 4FP | 4,00 |
| 3FP | 1,00 |
| 2FP | 2,65 |
| TFP | 0,71 |
| PFP | 2,62 |

On constate que c'est le 4FP qui donne le plus de sensibilité à la mesure.

On a effectué des essais avec des solutions de peroxydase autres que la peroxydase de raifort ; toutes ces solutions ont été positives. A ce sujet, on notera encore que la pureté d'une peroxydase telle que la peroxydase de raifort peut varier notablement suivant sa provenance. On traduit cette pureté par un nombre RZ (Reinheitzahl) rapporté à des mesures d'absorption optique

$$RZ = \frac{A\ 403\ nm}{A\ 275\ nm}$$

RZ peut atteindre la valeur 3 pour les produits de pureté maximum. A titre d'exemple, on donne ci-dessus, la valeur de RZ pour les peroxydases suivantes :

| | RZ |
|---|---|
| Peroxydase SIGMA | 0,64 |
| Peroxydase MILES LAB. | 1,25 |
| Peroxydase BOEHRINGER, Mannheim | 3,0 |

On notera encore que les procédures relatives à la mesure des autres co-enzymes à nicotinamide, notamment de NADPH et APADH dans les autres contextes ou ces co-enzymes sont impliqués sont identiques à ce qui a été décrit ci-dessus.

Exemple 6

Détermination de l'effet de la catalase

On a travaillé de manière similaire à ce qui est décrit dans les Exemples précédents et ajouté une quantité connue de solution de NADH a un milieu réactionnel préparé dans un tampon « tris », pH 7,4, 0,05 M. Le premier milieu réactionnel (A) a été préparé de manière à contenir une concentration en ions métalliques correspondant à la description du document EP-A 124 909 :

| | |
|---|---|
| $MN^{++}$ (sous forme de sulfate) | $10^{-2}$ M |
| AAP (4-aminoantipyrine) | $5 \times 10^{-4}$ M |
| POP (peroxydase) | 6 U/ml |
| NADH | $10^{-4}$ M |

On a laissé incuber ce mélange et après un temps déterminé : 10 sec (cas A1) et 2 min (cas A2), on a ajouté une quantité de p-fluorophénol (FP) telle que la solution soit 10 mM en ce réactif. On a alors enregistré la vitesse de libération de l'ion fluor comme décrit aux Exemples précédents.

On a répété ces essais dans des conditions identiques mais, cette fois, après avoir rajouté au milieu une quantité de catalase telle que celui-ci contienne 275 U/ml (cas AC1 et AC2).

On a préparé un second milieu réactionnel (B) inspiré des conditions de la présente invention :

| | |
|---|---|
| $MN^{++}$ | $10^{-5}$ M |
| AAP | — |
| POD | 2 U/ml |
| NADH | $10^{-3}$ M |

On a procédé comme ci-dessus avec la même quantité de p-fluorophénol ajouté après les mêmes temps de latence (B1) et (B2) et, dans un deuxième essai, avec 275 U/ml de catalase (cas BC1 et BC2).

Les vitesses de réaction obtenues (en unités arbitraires comparatives) figurent ci-dessous :

| Essai | Vitesse | Catalase |
|---|---|---|
| A1 | 7,1 | non |
| A2 | 7,6 | non |
| AC1 | 2,3 | oui |
| AC2 | 2,5 | oui |
| B1 | 3,0 | non |
| B2 | 3,0 | non |
| BC1 | 3,0 | oui |
| BC2 | 2,7 | oui |

On constate donc que, contrairement à ce qui se produit dans les cas (A), c'est-à-dire forte diminution de la formation de $H_2O_2$ en présence de catalase, cette dernière n'exerce qu'une influence négligeable sur la marche de la réaction suivant l'invention.

On notera par ailleurs que si, dans les cas A1 et A2 on opère dans un milieu privé d'aminoantipyrine (AAP) la vitesse réactionnelle est fortement ralentie, ce qui indique que cette substance joue un rôle d'activateur ; c'est exactement le contraire en ce qui concerne les cas B quoique, alors, la différence soit beaucoup moins nette. On peut donc dire que dans les conditions opératoires préconisées par le document EP-A-124 909, la détermination directe du $H_2O_2$ par simple remplacement de la méthode colorimétrique traditionnelle par la détermination fluorométrique du document EP-A-20 623 ne donne pas les résultats escomptés par une telle juxtaposition. Pour l'obtention de résultats optimalisés, il faut travailler en présence d'un activateur tel que AAP et en l'absence de catalase ou d'un quelconque autre facteur susceptible de consommer le $H_2O_2$ formé.

Exemple 7

Comparaisons entre le procédé de l'invention et celui du document EP-A-124 909

On a procédé dans les conditions générales de l'Exemple 6 en tampon « Tris » sur une prise de $2 \times 10^{-4}$ M de NADH, en présence de 6 U/ml de POD, en faisant varier le pH, la concentration en ions $Mn^{+2}$

et en présence ou non de 4-aminoantipyrine. Les conditions opératoires et les résultats sous forme des vitesses de réaction correspondantes (exprimées en µmole de $F^-$/l/min) sont rassemblés ci-dessous.

| Concentration $Mn^{+2}$ (M) | pH | 4-AAP | $[F^-]$ (µmole/l/min) |
|---|---|---|---|
| $10^{-2}$ | 7,5 | $5 \times 10^{-4}$ | 27 |
| $10^{-2}$ | 7,5 | 0 | 0,27 |
| $10^{-5}$ | 7,5 | $5 \times 10^{-4}$ | 0,04 |
| * $10^{-5}$ | 7,5 | 0 | 5,3 |
| $10^{-5}$ | 5,5 | $5 \times 10^{-4}$ | 7,2 |
| * $10^{-5}$ | 5,5 | 0 | 54,2 |

* Essais correspondant à l'invention.

Ces résultats montrent clairement les avantages de l'invention relativement à la référence : sensibilité plus grande à pH 5,5 ; effet inhibiteur de la 4-aminoantipyrine en présence d'un faible taux de manganèse, alors que c'est l'effet contraire qui se manifeste aux concentrations plus élevées de manganèse ($10^{-2}$ M), c'est-à-dire dans les conditions où la formation de $H_2O_2$ prédomine.

Exemple 8

Performance comparative de divers composés aromatiques fluorés à pH 5,5

On a procédé par analogie avec les Exemples 2 et 5 en utilisant les réactifs suivants :

a) Tampon cacodylate : comme à l'Exemple 2, 0,1 M, pH 5,5.

b) Milieu réactionnel : on dissout 0,425 µmole du composé fluoré cité ci-dessous dans environ 40 ml de tampon (a), on ajoute 0,2 ml de solution de NaF $10^{-3}$ M et on complète à 50 ml avec le tampon (a). Concentration en $F^- = 4 \times 10^{-6}$ M. Les composés fluorés suivants ont été utilisés :

b1 4-fluorophénol (56 mg) 4-FP ;
b2 4-fluoroaniline (55,5 mg) 4-FA ;
b3 tetrafluorophénol (83 mg) TFP ;
b4 pentafluorophénol (92 mg) PFP.

c) Solution de $MnCl_2$ à $10^{-3}$ M dans de l'eau bidistillée.

d) Solution de peroxidase (POD) à 600 U/ml dans un tampon acétate 0,01 M à pH 5,5 (peroxydase SIGMA à 100 U/mg).

e) Solution de NADH $5 \times 10^{-2}$ M dans un tampon « Tris » 0,05 M à pH 7,5.

Pour la mesure, on a procédé comme suit : Dans un bécher de polyéthylène (10 ml) on a placé 4,85 ml de la solution (b), 0,05 ml de la solution (c) et 0,05 ml de la solution de peroxydase (d). On a plongé l'électrode à fluor dans la solution agitée magnétiquement, on a laissé l'appareil se stabiliser 1 min, puis on a ajouté 0,05 ml de la solution de NADH (e).

S'agissant d'une mesure de type comparatif, on s'est contenté dans ce test de mesurer la quantité de $F^-$ libérée 1 minute après l'adjonction de la solution (e) (technique de mesure dite « à temps fixé »).

Les résultats réunis au tableau ci-dessous en fonction de la nature du composé fluoré utilisé montrent qu'à pH 5,5 c'est le PFP qui fournit les tests les plus sensibles.

| Composé fluoré | $F^-$ (µm) |
|---|---|
| 4-FP | 17,9 |
| 4-FA | 9,1 |
| TFP | 57,7 |
| PFP | 62,7 |

**Revendications**

1. Procédé analytique pour la détermination d'un co-enzyme à nicotinamide réduit, notamment NADH, NADPH et APADH, caractérisé par le fait qu'on met celui-ci en présence d'un composé aromatique

à noyau fluoré, d'oxygène, d'une substance à activité peroxidasique et, facultativement, d'ions $Mn^{+2}$ ou d'autres ions métalliques en concentration ne dépassant pas $10^{-4}$ M, ledit co-enzyme ayant alors la propriété de provoquer la séparation de l'atome de fluor de celui-ci en fluorure, puisqu'on dose l'ion fluorure ainsi formé, la vitesse de formation de celui-ci étant quantitativement liée à la quantité originale de co-enzyme à déterminer.

2. Procédé suivant la revendication 1, caractérisé par le fait que le milieu réactionnel comprend au moins un constituant additionnel réagissant en présence dudit co-enzyme à nicotinamide ou de la forme oxydée de celui-ci, la mise en œuvre du procédé permettant de doser ce constituant additionnel.

3. Procédé suivant la revendication 1, caractérisé par le fait qu'on dose électrométriquement les ions fluorures au moyen d'une électrode spécifique de ces ions.

4. Procédé suivant la revendication 3, caractérisé par le fait qu'on travaille dans un tampon à pH 5-7,5.

5. Procédé suivant la revendication 4, caractérisé par le fait que le tampon est un tampon « tris » ou acétate.

6. Procédé suivant la revendication 4, caractérisé par le fait qu'on travaille dans un tampon cacodylate de molarité de 0,05 M à 0,5 M, la vitesse de réaction dépendant de la concentration en diméthylarsinate de ce tampon.

7. Procédé suivant la revendication 4, caractérisé par le fait qu'on ajoute au milieu d'analyse des ions $Mn^{++}$ en concentration de $2 \times 10^{-6}$-$10^{-4}$ M.

8. Procédé suivant la revendication 2, caractérisé par le fait qu'on l'applique au dosage du glucose dans des échantillons de fluides biologiques, celui-ci réagissant avec l'ATP en présence d'hexokinase pour donner du glucose-6-phosphate et ce dernier étant oxydé par le $NAD^+$ en présence de glucose-6-phosphate déshydrogenase en D-gluconate-6-phosphate avec formation correspondante de NADH en proportion directe du glucose à mesurer.

9. Procédé suivant la revendication 2, caractérisé par le fait qu'on l'applique au dosage de l'urée dans les fluides biologiques, celle-ci fournissant de l'ammoniaque par hydrolyse en présence d'uréase, cet ammoniaque fournissant du L-glutamate par réaction avec le 2-oxoglutorate en présence de glutamate déshydrogenase et de NADH, la proportion consommée de celui-ci étant en raison directe de l'urée à mesurer.

10. Procédé suivant la revendication 3, caractérisé par le fait qu'on utilise, comme composé à noyau fluoré, un composé choisi parmi les 2-, 3-, et 4-fluorophénols, le tetrafluorophénol, le pentafluorophénol et la 4-fluoroaniline.

11. Procédé suivant la revendication 1, caractérisé en ce que la substance à activité peroxidasique est choisie parmi l'enzyme peroxydase et l'hémoglobine et ses dérivés.

**Claims**

1. An analytical method for determining a co-enzyme containing reduced nicotinamide, inter alia NADH, NADPH an APADH, characterised in that the co-enzyme is placed in the presence of a fluorinated-ring aromatic compound, oxygen, a substance having peroxidase activity and, optionally, $Mn^{+2}$ ions or other metal ions in concentration not above $10^{-4}$ M, the co-enzyme being then under conditions suitable to separate the fluoride atom therefrom into fluoride after which the resulting fluoride ion is determined, the rate at which it is formed being quantitatively linked to the original quantity of co-enzyme to be determined.

2. A method according to claim 1, characterised in that the reaction medium comprises at least one additional constituent reacting in the presence of said nicotinamide containing co-enzyme or the oxidised form thereof, the method being adapted for determining the additional constituent.

3. A method according to claim 1, characterised in that the fluoride ions are determined electrometrically by means of an electrode which is specific to these ions.

4. A method according to claim 3, characterised in that a buffer at pH 5-7.5 is used.

5. A method according to claim 4, characterised in that the buffer is « tris » or acetate.

6. A method according to claim 4, characterised in that a cacodylate buffer is used at a molar concentration of 0.05 to 0.5 M, the reaction rate depending on the concentration of dimethylarsinate in the buffer.

7. A method according to claim 4, characterised in that $Mn^{++}$ ions in a concentration of $2 \times 10^{-6}$-$10^{-4}$ M are added to the analytical medium.

8. A method according to claim 2, characterised in that it is used for determining glucose in samples of biological fluids, the glucose reacting with ATP in the presence of hexokinase to give glucose-6-phosphate which is oxydised by $NAD^+$ in the presence of glucose-6-phosphate with corresponding production of NADH in direct proportion to the glucose to be measured.

9. A method according to claim 2, characterised in that it is used for determining urea in biological fluids, which urea will supply ammonia by hydrolysis in the presence of urease, and the said ammonia will supply L-glutamate by reaction with 2-oxoglutarate in the presence of glutamate dehydrogenase and NADH, the consumption of the latter being in direct proportion to the urea to be measured.

10. A method according to claim 3, characterised in that the fluorinated-ring compound used is chosen from among 2-, 3- and 4-fluorophenol, tetrafluorophenol, pentafluorophenol and 4-fluoroaniline.

11. A method according to claim 1, characterised in that the substance with peroxidase activity is selected from the enzyme peroxidase, hemoglobin and derivatives thereof.

**Patentansprüche**

1. Analyseverfahren zur Bestimmung eines zu Nicotinamid reduzierten Coenzyms, insbesondere NADH, NADPH und APADH, dadurch gekennzeichnet, daß man dieses in Gegenwart einer aromatischen, im Ring Fluor enthaltenden Verbindung, Sauerstoff, einer peroxidisch wirkenden Substanz und wahlweise $Mn^{2+}$-Ionen oder anderer Metallionen bei einer $10^{-4}$ M nicht überschreitenden Konzentration ausführt, wobei das Coenzym dann die Eigenschaft hat, die Abtrennung des Fluoratoms aus der Fluorverbindung zu provozieren, worauf man das so gebildete Fluoridion mißt, wobei die Geschwindigkeit seiner Ausbildung quantitativ der ursprünglichen Menge des zu bestimmenden Coenzyms entspricht.

2. Verfahren nach Anspruch 1, dadurch gekennzeichnet, daß das Reaktionsmedium mindestens einen weiteren Bestandteil enthält, der in Gegenwart des Coenzyms zu Nicotinamid oder seiner oxidierten Form reagiert, wobei die Arbeitsweise das Messen dieses zusätzlichen Bestandteils erlaubt.

3. Verfahren nach Anspruch 1, dadurch gekennzeichnet, daß man die Fluoridionen mittels einer für diese Ionen spezifischen Elektrode elektronisch mißt.

4. Verfahren nach Anspruch 3, dadurch gekennzeichnet, daß man in einer Pufferlösung bei pH 5 bis 7,5 arbeitet.

5. Verfahren nach Anspruch 4, dadurch gekennzeichnet, daß der Puffer ein Tris-Puffer oder ein Acetat ist.

6. Verfahren nach Anspruch 4, dadurch gekennzeichnet, daß man in einer Cacodylatpufferlösung mit einer Molarität von 0,05 M bis 0,5 M arbeitet, wobei die Reaktionsgeschwindigkeit von der Dimethylarsinatkonzentration dieser Pufferlösung abhängt.

7. Verfahren nach Anspruch 4, dadurch gekennzeichnet, daß man dem Analysemedium $Mn^{2+}$-Ionen in einer Konzentration von $2 \times 10^{-6}$ bis $10^{-4}$ M zufügt.

8. Verfahren nach Anspruch 2, dadurch gekennzeichnet, daß man in der Probe der biologischen Flüssigkeiten eine Menge an Glukose einsetzt, die mit ATP in Gegenwart von Hexokinase unter Bildung von Glukose-6-phosphat reagiert und die schließlich durch das $NAD^+$ in Gegenwart von Glukose-6-phosphatdehydrogenase in D-Glukonat-6-phosphat unter entsprechender Ausbildung von NADH in direkter Proportion zu der zu messenden Glukose oxidiert wird.

9. Verfahren nach Anspruch 2, dadurch gekennzeichnet, daß man eine Menge an Harnstoff in den biologischen Flüssigkeiten einsetzt, der durch Hydrolyse in Gegenwart von Urease Ammoniak liefert, wobei dieser Ammoniak durch Reaktion mit 2-Oxoglutorat in Gegenwart von Glutamatdehydrogenase und von NADH L-Glutamat liefert, wobei der Anteil an dem Verbrauch desselben in direktem Verhältnis zu dem zu messenden Harnstoff steht.

10. Verfahren nach Anspruch 3, dadurch gekennzeichnet, daß man als im Ring Fluor enthaltende Verbindung eine Verbindung aus der Gruppe der 2-, 3- und 4-Fluorphenole, Tetrafluorphenol, Pentafluorphenol und 4-Fluoranilin verwendet.

11. Verfahren nach Anspruch 1, dadurch gekennzeichnet, daß man als peroxidisch wirkende Substanz das Peroxidaseenzym und Hämoglobin und seine Derivate auswählt.

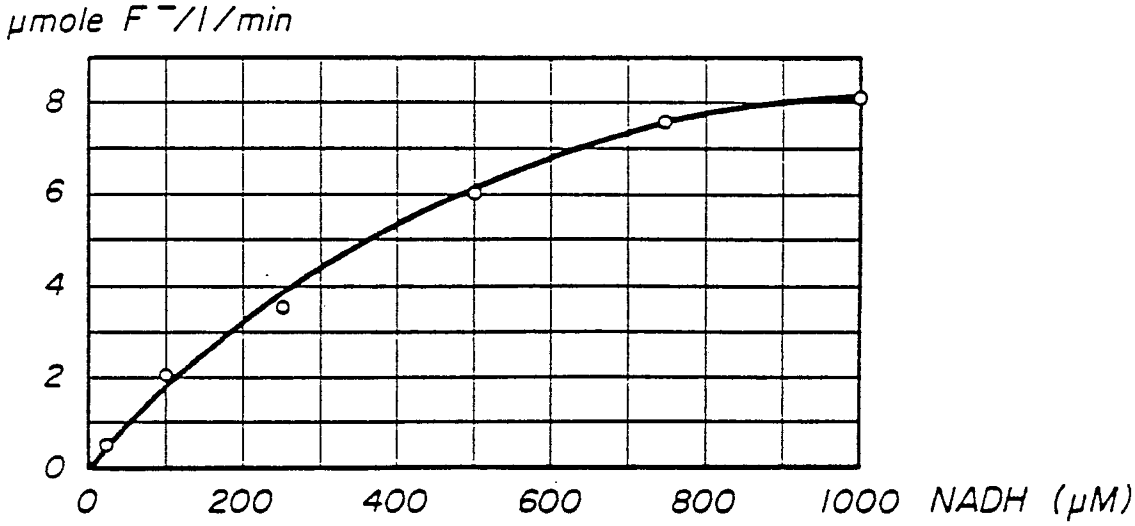


FIG. 1

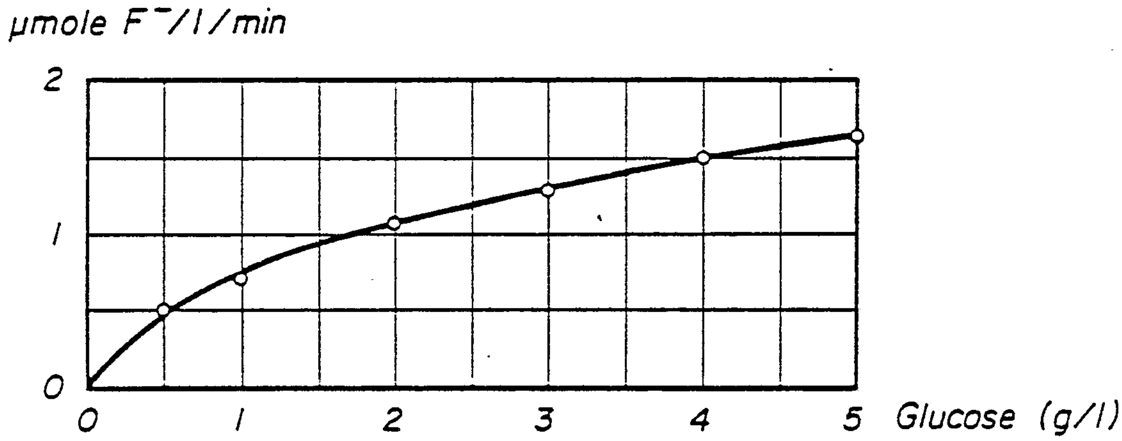


FIG. 2

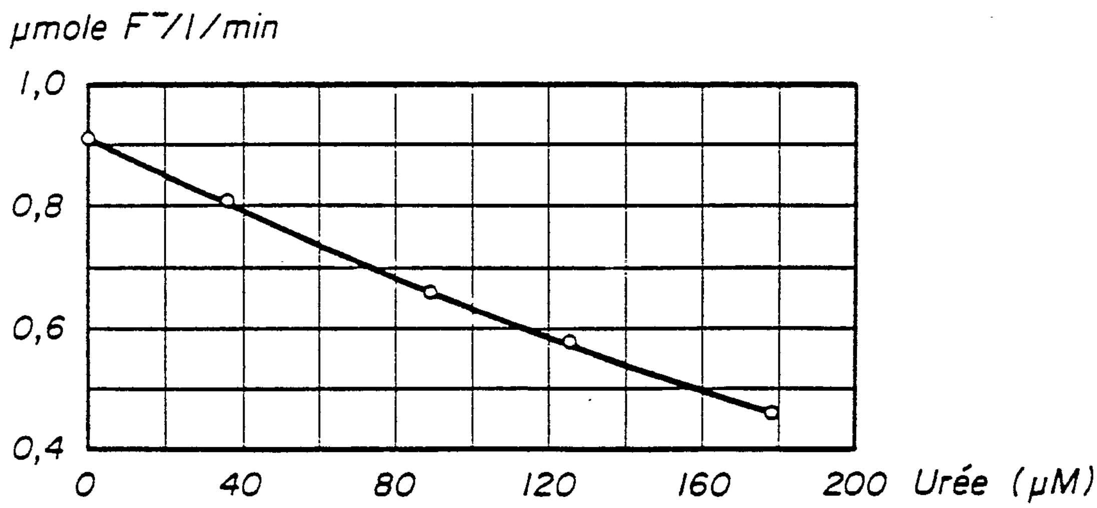


FIG. 3

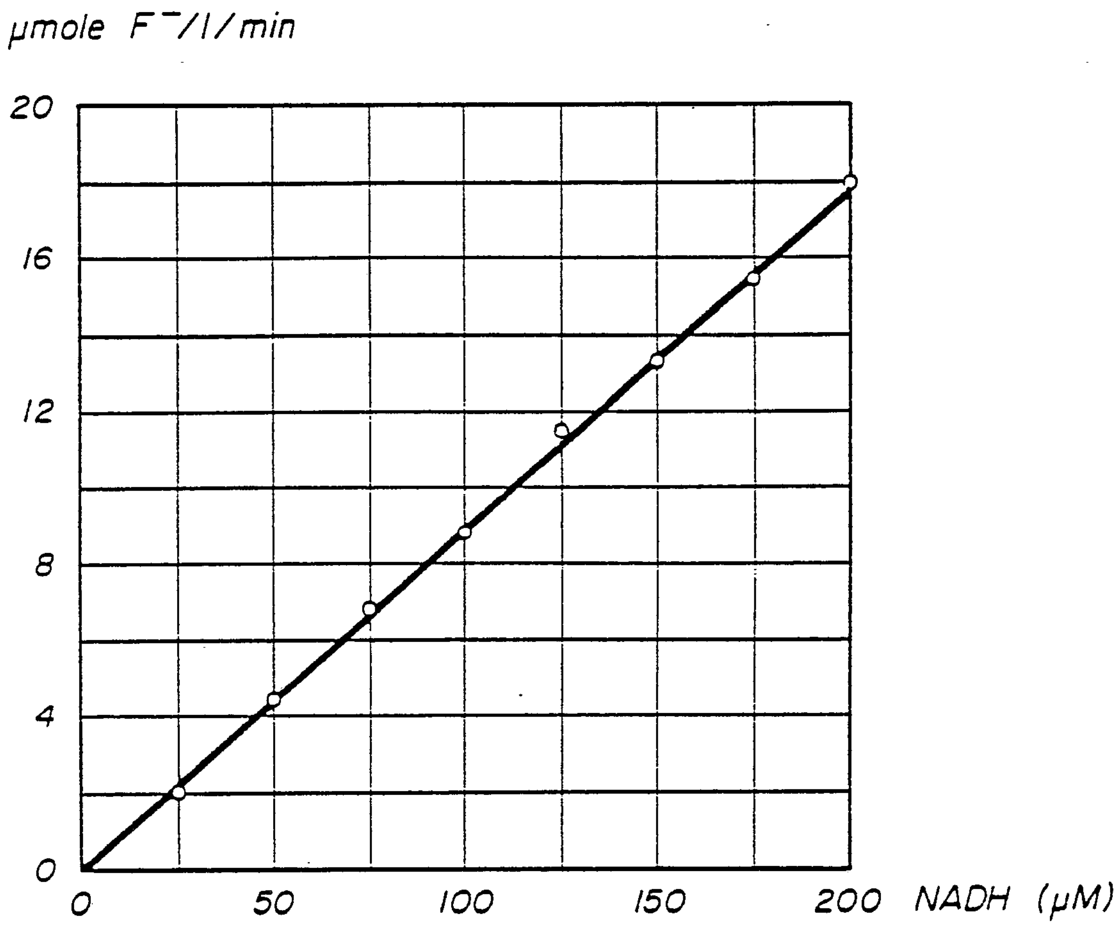


FIG. 4